(19) **Europäisches Patentamt**

**European Patent Office**

**Office européen des brevets**

(11) **EP 1 693 001 A1**

(12) **EUROPEAN PATENT APPLICATION**

(43) Date of publication:
**23.08.2006 Bulletin 2006/34**

(51) Int Cl.:
**A61B 5/022** (2006.01)

(21) Application number: **06002570.7**

(22) Date of filing: **08.02.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **14.02.2005 JP 2005036066**

(71) Applicant: **Omron Healthcare Co., Ltd.**
**Kyoto 615-0084 (JP)**

(72) Inventors:
• **Sano, Yoshihiko**
c/o Omron Healthcare Co.,Ltd.
**Kyoto 615-0084 (JP)**

• **Kishimoto, Hiroshi**
c/o Omron Healthcare Co.,Ltd.
**Kyoto 615-0084 (JP)**
• **Karo, Hiromichi**
c/o Omron Healthcare Co.,Ltd.
**Kyoto 615-0084 (JP)**
• **Tsurumi, Yoshinori**
c/o Omron Healthcare Co.,Ltd.
**Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(54) **Blood pressure monitor and corresponding cuff**

(57) An air bag (150) being inflated and deflated as air comes in and out is included. The air bag (150) is formed of a flexible sheet material. The air bag (150) has at least an inner circumferential sheet (162) positioned inside when a blood pressure monitor cuff is wrapped around a living body, and an outer circumferential sheet (161) positioned on the outer circumferential side of the inner circumferential sheet (162). The sheet material forming the air bag (150) has a thickness of 0.15 mm or less. Therefore, big deep wrinkles appearing on the inner surface of the fluid bag fitted on a living body can be reduced, which may otherwise give an adverse effect on blood pressure measurement.

FIG.1

FIG.2

FIG.3

FIG.4

FIG.5

2

# FIG.6

150
161    166a
151
152
153
154
162    165    166b    164

FIG.7

α2    α1    t
150
162
161
r
L2
L1

FIG.8

R
150
140

FIG.9

C
R
150
140

FIG.10

AIR BAG MATERIAL : STRETCH PU, t=0.2 mm

(a)

(b)

FIG.11

AIR BAG MATERIAL : STRETCH PU, t=0.15 mm

(a)

(b)

FIG.12

AIR BAG MATERIAL : STRETCH PU, t=0.1 mm

(a)

(b)

FIG.13

4

FIG.14

## Description

BACKGROUND OF THE INVENTION

Field of the Invention

The present invention relates to a blood pressure monitor cuff with an air bag for squeezing a living body to interrupt the blood flow through an artery and a blood pressure monitor including the same.

Description of the Background Art

Usually, in order to measure blood pressure, a cuff containing a fluid bag for squeezing an artery in a living body is first wrapped around the surface of the living body. Pressure is then applied to the wrapped fluid bag and reduced as appropriate, so that an artery pulse pressure and wave in the artery is detected. The blood pressure is thus measured.

Here, a cuff refers to a hollow strap-like structure that can be wrapped around a part of a living body. Fluid such as gas or liquid is injected into the cuff in order to measure an artery pulse pressure of the arm or leg of a living body. Therefore, a cuff implies the concept including a fluid bag and wrapping means for wrapping the fluid bag around a living body. In particular, a cuff wrapped around and fitted on the wrist or upper arm of a human body is sometimes called an armband or a manchette.

A fluid bag used in such a cuff is formed by two-dimensionally joining a pair of flexible sheet materials formed of a resin or the like and welding the peripherals thereof together. The fluid bag formed in this manner is wrapped around the upper arm or wrist of a human body and a fluid is injected into the fluid bag to apply pressure. Here, the difference in circumferential length between an inner circumferential sheet and an outer circumferential sheet forming the fluid bag causes slacks and wrinkles on the inner peripheral sheet.

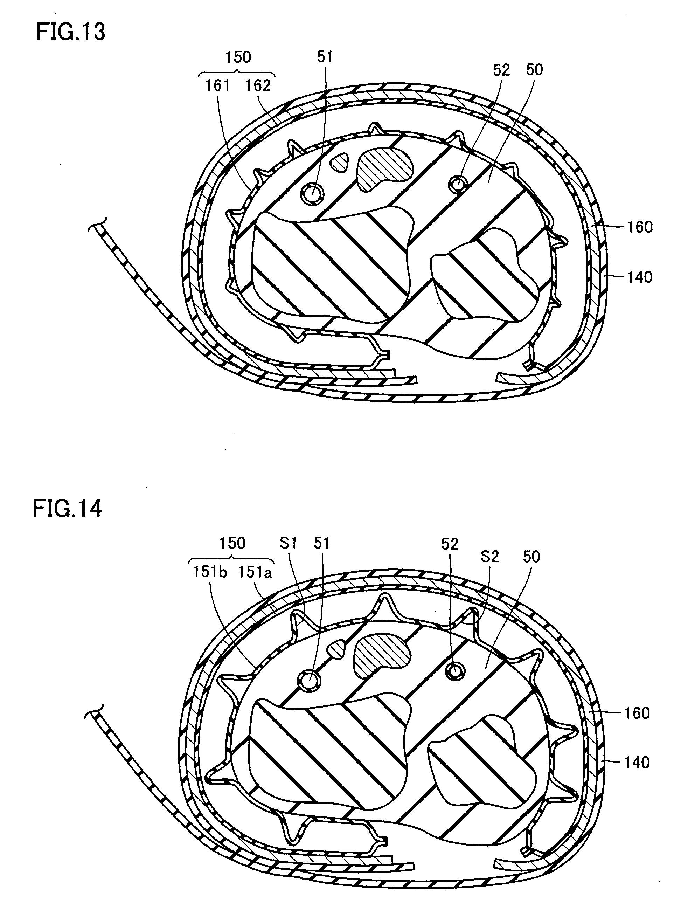

The position of an artery varies for each subject of measurement because of variations among individual living bodies. For reliable squeeze, the fluid bag wrapped around a living body is preferably inflated evenly along its entire length. However, when the above-noted wrinkle appears above the artery to be squeezed, the wrinkle obstructs sufficient inflation of the fluid bag. Thus, the artery may be squeezed insufficiently. This state will be described in detail with reference to Fig. 14.

Fig. 14 shows a cross section of a blood pressure monitor cuff fitted on the wrist. In Fig. 14, an air bag 150 is fitted on a wrist portion 50 such that its outer circumferential surface is restrained by a curled elastic member 160 and a cover body 140. Arteries 51 and 52 existing in wrist portion 50 need to be squeezed sufficiently by air bag 150. However, big wrinkles S1 and S2 at an inner circumferential sheet 151 cause spaces above arteries 51 and 52, so that arteries 51 and 52 are not squeezed sufficiently. In such a case, air bag 150 cannot interrupt the blood flow through arteries 51 and 52 sufficiently. Therefore, the artery pulse pressure and wave cannot be detected correctly, thereby possibly degrading the measurement precision or disabling the measurement.

In particular, in a wrist blood pressure monitor, a radius of curvature of a fluid bag fitted on the wrist is smaller as compared with an upper arm blood pressure monitor. Therefore, the difference in circumferential length between the outer circumferential sheet and the inner circumferential sheet has a significant impact on the inner circumferential sheet, so that big wrinkles are likely to appear on the inner circumferential sheet.

Japanese Patent Laying-Open No. 62-072315 discloses a blood pressure monitor cuff including an air bag as described above. In this document, the outer peripheries of a pair of sheet materials are welded together in the shape of a bag. In addition, joint portions formed by welding these sheet materials together are provided at appropriate intervals inside the fluid bag. Provision of joint portions at appropriate intervals encourages wrinkles to be formed at locations provided with the joint portions and prevents deep wrinkles.

In the blood pressure monitor cuff disclosed in Japanese Patent Laying-Open No. 62-072315, the joint portions are provided by joining the inner circumferential sheet and the outer circumferential sheet together at appropriate intervals, thereby to encourage wrinkles to be formed at the joint portions. However, the upper arm or wrist wearing a cuff varies in size and shape among individuals. For example, there is a considerable difference between a relatively big adult male and a small female or a child. Therefore, if a female or child uses a cuff having an interval between joint portions that is set relatively wide based on an adult male, deep wrinkles are rather formed at the joint portions, and these wrinkles may prevent arteries from being squeezed sufficiently. In addition, these wrinkles divide the fluid bag, so that the fluid is not distributed over the entire fluid bag and the inflation of the fluid bag becomes uneven. As a result, arteries may not be squeezed stably. On the other hand, if an adult male uses a cuff having an interval between joint portions that is set relatively narrow based on a relatively small female or a child, a sufficient thickness cannot be secured for the fluid bag, so that the arteries may not be squeezed sufficiently.

Moreover, if the position of a wrinkle is fixed by providing a joint portion as described above, the position of the artery may correspond to the position of the fixed wrinkle in some subjects. In such a case, the artery cannot be squeezed stably, which may adversely affect the precision in measurement.

SUMMARY OF THE INVENTION

It is an object of the present invention to provide a blood pressure monitor cuff and a blood pressure monitor, in which big deep wrinkles in the inner face of the fluid bag of the cuff fitted on a living body can be prevented.

A blood pressure monitor cuff in accordance with the present invention includes a fluid bag being inflated and deflated as a fluid comes in and out. The fluid bag is formed of a flexible sheet material. The fluid bag has at least an inner circumferential sheet positioned inside when the blood pressure monitor cuff is wrapped around a living body, and an outer circumferential sheet positioned on the outer circumferential side of the inner circumferential sheet. The sheet material forming the fluid bag has a thickness of 0.15 mm or less.

The present invention is made based on a completely new knowledge over prior arts in that a fluid bag is formed of a sheet material thinner than a conventionally used sheet material, so that a difference in circumferential length between an outer circumferential sheet and an inner circumferential sheet is reduced when the blood pressure monitor cuff is wrapped around a living body, thereby reducing the influence of the wrinkle formed on the inner circumferential sheet.

This knowledge will now be described in detail. A certain thickness is required for a fluid layer in order to absorb protrusions and depressions on the surface of a living body and to allow a fluid to flow smoothly in the fluid layer inside the fluid bag. However, the thickness of the sheet material forming the fluid bag is essentially not limited, as long as the sheet material can hold the fluid layer. Then, in the present invention, the thickness of the sheet material of the fluid bag for forming the fluid layer is reduced, so that the entire thickness of the fluid bag is reduced while the thickness of the fluid layer is kept equal to the conventional one. Accordingly, when this fluid bag is wrapped around a living body, the difference in circumferential length between the outer circumferential sheet and the inner circumferential sheet can be reduced. As a result, the slacks on the inner circumferential sheet can be reduced and the big deep wrinkles appearing on the inner circumferential sheet can be decreased, thereby allowing stable squeeze on arteries.

In the present invention, the inventors conducted an elaborate study to find that the effect as described above is sufficiently brought about when the thickness of the sheet material forming the fluid bag is equal to or less than 0.15 mm. With the thickness of the sheet material equal to or less than 0.15 mm, the difference in circumferential length between the outer circumferential sheet and the inner circumferential sheet can be reduced enough when the fluid bag is wrapped around a living body, thereby surely reducing big deep wrinkles formed on the inner circumferential sheet. As a result, the fluid bag can squeeze an artery stably.

In the blood pressure monitor cuff as described above, the fluid bag may have a plurality of fluid layers radially overlapping with each other when the blood pressure monitor cuff is wrapped around a living body. When the fluid bag has a plurality of fluid layers, the number of sheet materials increases. Thus, wrinkles appearing on the inner circumferential sheet may be a problem. However, the reduced thickness of the sheet material can effectively prevent deep big wrinkles.

In the blood pressure monitor cuff as described above, the sheet material forming the fluid bag may be made of polyurethane. When the sheet material forming the fluid bag is reduced in thickness, its strength should be ensured. In particular, the strength in the welding portion of the sheet material is important. Using the sheet material made of polyurethane, sufficient strength can be secured particularly at the welding portion even when the thickness is reduced.

As described above, in a wrist blood pressure monitor, wrinkles tend to adversely affect the measurement of blood pressure due to a small radius of curvature of a wrist portion. However, when the blood pressure monitor cuff as described above is used for a wrist blood pressure monitor, big deep wrinkles can be reduced effectively, thereby avoiding any inconvenience in measurement caused by wrinkles.

A blood pressure monitor may be formed of the blood pressure monitor cuff as described above with provision of an inflation/deflation unit inflating and deflating the fluid bag, a pressure detection unit detecting a pressure in the fluid bag, and a blood pressure value calculation unit calculating a blood pressure value based on information of the pressure detected by the pressure detection unit.

In the blood pressure monitor cuff and the blood pressure monitor using the same in accordance with the present invention, big deep wrinkles can be reduced on the inner face of the fluid bag fitted on a living body. Therefore, an artery can be squeezed reliably, and blood pressure can be measured stably.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is an external perspective view of a blood pressure monitor in an embodiment in accordance with the present invention.
Fig. 2 is a cross sectional view showing an interior structure of a blood pressure monitor cuff shown in Fig. 1.
Fig. 3 is a block diagram showing a configuration of the blood pressure monitor in an embodiment in accordance with the present invention.
Fig. 4 is a flowchart showing a flow of a blood pressure measurement process in an embodiment in accordance with the present invention.
Fig. 5 is a perspective view, partially broken away, showing an air bag in an embodiment in accordance with the present invention.

Fig. 6 is a cross sectional view taken along line VI-VI viewed in the direction of arrows in Fig. 5, showing the air bag in an embodiment in accordance with the present invention.

Fig. 7 is an illustration showing each dimension of a fluid bag for use in calculation for verifying the effect of the present invention.

Fig. 8 is an illustration showing an experimental method of verifying the effect of the present invention.

Fig. 9 is an illustration showing an experimental method of verifying the effect of the present invention.

Fig. 10 shows an experimental result in a comparative example, in which (a) shows a photograph of an inner circumferential surface of an air bag and (b) is an illustration based on the photograph showing wrinkles on the inner circumferential surface of the air bag.

Fig. 11 shows an experimental result in an example in accordance with the present invention, in which (a) shows a photograph of an inner circumferential surface of an air bag and (b) is an illustration based on the photograph showing wrinkles on the inner circumferential surface of the air bag.

Fig. 12 shows an experimental result in an example in accordance with the present invention, in which (a) shows a photograph of an inner circumferential surface of an air bag and (b) is an illustration based on the photograph showing wrinkles on the inner circumferential surface of the air bag.

Fig. 13 is a cross sectional view showing that a blood pressure monitor cuff of an embodiment in accordance with the present invention squeezes arteries.

Fig. 14 is a cross sectional view showing that a conventional blood pressure monitor cuff squeezes arteries.

DESCRIPTION OF THE PREFERRED EMBODIMENT

In the following, an embodiment of the present invention will be described in detail with reference to the figures. It is noted that although in the following embodiment a wrist blood pressure monitor will be described by way of illustration, the blood pressure monitor cuff and the blood pressure monitor in accordance with the present invention is not limited to a wrist blood pressure monitor and may be applied to other blood pressure monitors such as an upper arm blood pressure monitor.

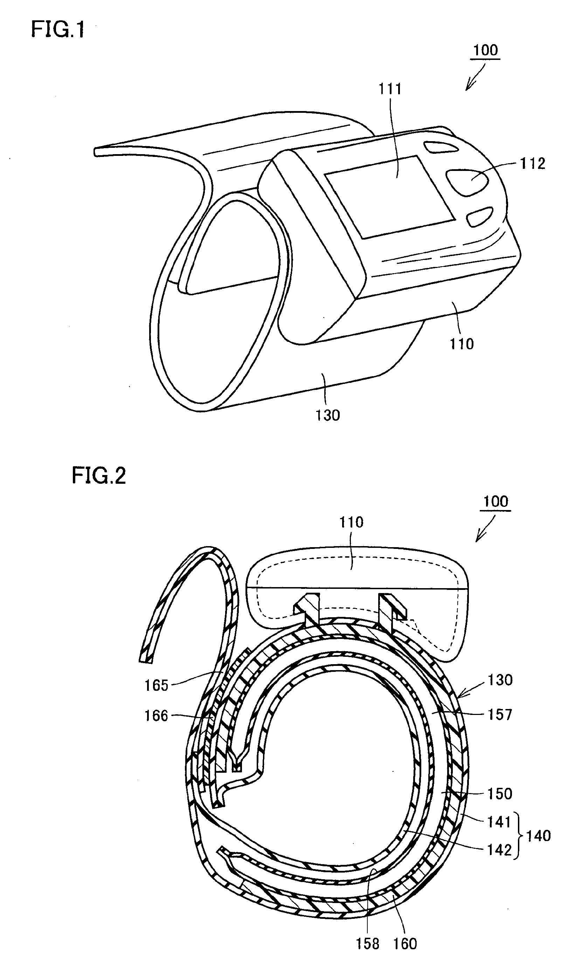

Fig. 1 is an external perspective view of a blood pressure monitor in an embodiment of the present invention. As shown in Fig. 1, a blood pressure monitor 100 in the embodiment of the present invention includes an apparatus body 110 and a cuff 130. A display unit 111 and an operation unit 112 are arranged on the surface of apparatus body 110, and the cuff 130 is attached to the apparatus body 110.

Fig. 2 is a cross sectional view showing the interior structure of the blood pressure monitor cuff shown in Fig. 1. As shown in Fig. 2, blood pressure monitor cuff 130 in this embodiment essentially includes a bag-like cover body 140 formed of a cloth or the like, an air bag 150 as a fluid bag arranged inside the cover body 140, and a curled elastic member 160 having bending elasticity, which is arranged in the interior of cover body 140 and positioned outside air bag 150 in a fitted state for temporarily fitting the cuff on the wrist. These cover body 140, air bag 150 and curled elastic member 160 extend longitudinally with respect to the direction in which cuff 130 is wrapped.

Cover body 140 includes an inner cover 142 positioned inside in a fitted state and formed of a highly stretchy cloth or the like, and an outer cover 141 positioned outside of inner cover 142 and formed of a less stretchy cloth or the like. These inner cover 142 and outer cover 141 are superposed and stitched together at their peripheries thereby to form a bag. A hook-and-loop fastener 165 is provided on the inner circumferential surface side of cover body 140 at one end thereof in the longitudinal direction. A hook-and-loop fastener 166 engaging the hook-and-loop fastener 165 is affixed on the outer circumferential surface of cover body 140 on the other end thereof in the longitudinal direction. These hook-and-loop fasteners 165, 166 are means for stably fixing blood pressure monitor 100 on the wrist.

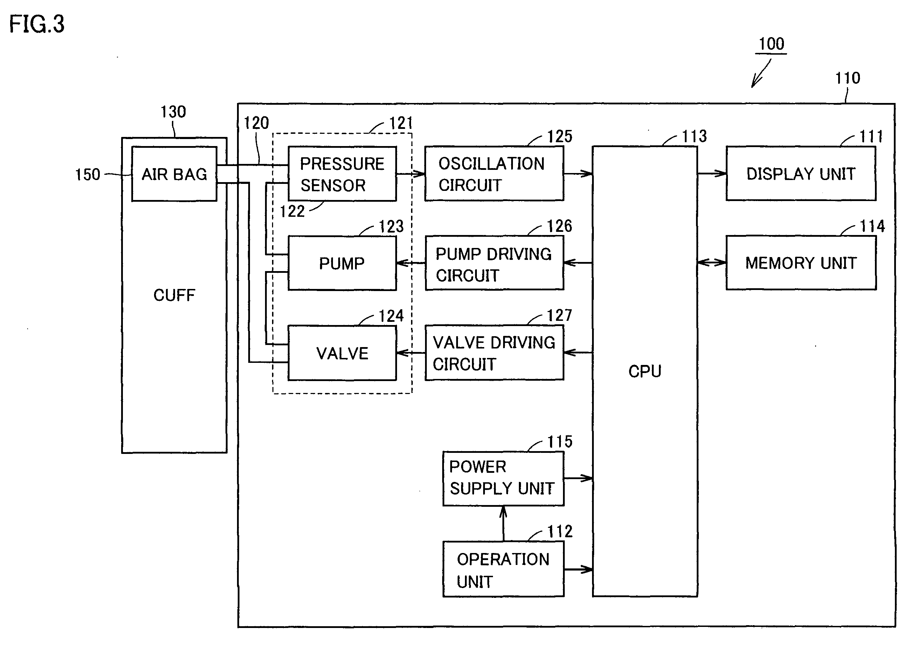

Air bag 150 is formed of a bag-like member formed of a resin sheet as a sheet material and includes an inflation/deflation space 157 therein. The inner circumferential surface of air bag 150 functions as a squeezing surface 158 for squeezing the wrist. Inflation/deflation space 157 is connected to an air system 121 for blood pressure measurement of apparatus body 110 described later through a tube 120 (see Fig. 3). The detailed structure of air bag 150 will be described later.

Curled elastic member 160 is arranged outside air bag 150. Curled elastic member 160 is formed of a flexible member elastically deformable in the radial direction when being annually wound around. Curled elastic member 160 is adhered to the outer surface of air bag 150 by not-shown adhesion means such as a double-sided tape. The curled elastic member 160 is formed in such a manner as to keep its annular shape and acts such that air bag 150 tightly fits on a living body in a fitted state. The curled elastic member 160 is formed, for example, of a resin member such as polypropylene so as to develop sufficient elasticity.

Fig. 3 is a block diagram showing the configuration of the blood pressure monitor in this embodiment. As shown in Fig. 3, apparatus body 110 includes an air system 121 blood pressure measurement for supplying or exhausting air to/from the air bag 150 through a tube 120, and an oscillation circuit 125, a pump driving circuit 126 and a valve driving circuit 127 provided in connection with air system 121 for blood pressure measurement.

These components function as an inflation/deflation unit for inflating and deflating air bag 150.

Apparatus body 110 also includes a CPU (Central Processing Unit) 113 for intensively controlling and watching each unit, a memory unit 114 for storing a program causing CPU 113 to execute prescribed operations and a variety of information such as a measured blood pressure value, a display unit 111 for displaying a variety of information including a blood pressure measurement result, an operation unit 112 operated to input a variety of instructions for measurement, and a power supply unit 115 for supplying power to CPU 113 in response to a power-on instruction from operation unit 112. CPU 113 functions as blood pressure value calculation means for calculating a blood pressure value.

Air system 121 for blood pressure measurement includes a pressure sensor 122 for measuring a pressure in air bag 150 (referred to as "cuff pressure" hereinafter), a pump 123 for supplying air to air bag 150, and a valve 124 opened and closed for exhausting or introducing air for air bag 150. Pressure sensor 122 functions as pressure detection means for detecting the cuff pressure. Oscillation circuit 125 outputs to CPU 113 a signal of an oscillation frequency according to an output value from pressure sensor 122. Pump driving circuit 126 controls driving of pump 123 based on a control signal applied from CPU 113. Valve driving circuit 127 controls opening and closing of valve 124 based on a control signal applied from CPU 113.

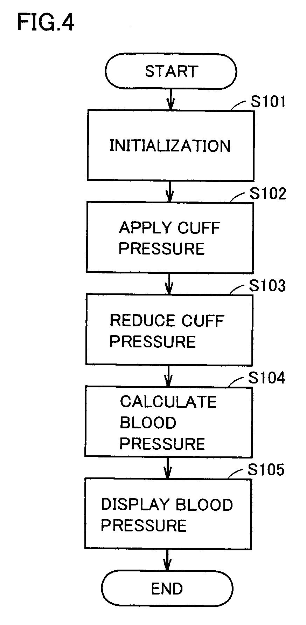

Fig. 4 is a flowchart showing a flow of a blood pressure measurement process in the blood pressure monitor in this embodiment. The program according to this flowchart is stored beforehand in memory unit 114. CPU 113 reads and executes the program from memory unit 114 to perform a blood pressure measurement process.

As shown in Fig. 4, when a subject operates an operation button of operation unit 112 of blood pressure monitor 100 for power-on, blood pressure monitor 100 is initialized (step S101). Then, upon reaching a measurable state, CPU 113 starts driving pump 123 and gradually increases the cuff pressure of air bag 150 (step S102). In the process of gradually applying pressure, when the cuff pressure reaches a prescribed level for blood pressure measurement, CPU 113 stops pump 123 and then gradually opens the originally closed valve to gradually exhaust air from air bag 150 and gradually decrease the cuff pressure (step S103). In this embodiment, a blood pressure is measured in the process of slowly decreasing the cuff pressure.

Then, CPU 113 calculates a blood pressure (a systolic blood pressure value and a diastolic blood pressure value) in a well-known procedure (step S104). Specifically, in the process of gradually decreasing the cuff pressure, CPU 113 extracts pulse wave information based on an oscillation frequency obtained from oscillation circuit 125. Then, a blood pressure value is calculated based on the extracted pulse wave information. When a blood pressure value is calculated at step S104, the calculated blood pressure value is displayed on display unit 111 (step S105). It is noted that the measurement scheme as described above is based on a so-called depressurization measurement scheme in which a pulse wave is detected during depressurization of the air bag. Alternatively, a so-called pressurization measurement scheme may also be employed, as a matter of course, in which a pulse wave is detected during pressurization of the air bag.

Blood pressure monitor 100 and blood pressure monitor cuff 130 in this embodiment are characterized by the structure of air bag 150 arranged in blood pressure monitor cuff 130. In the following, the structure of air bag 150 will be described in detail with reference to the figure. Here, an air bag having two air layers will be described by way of example.

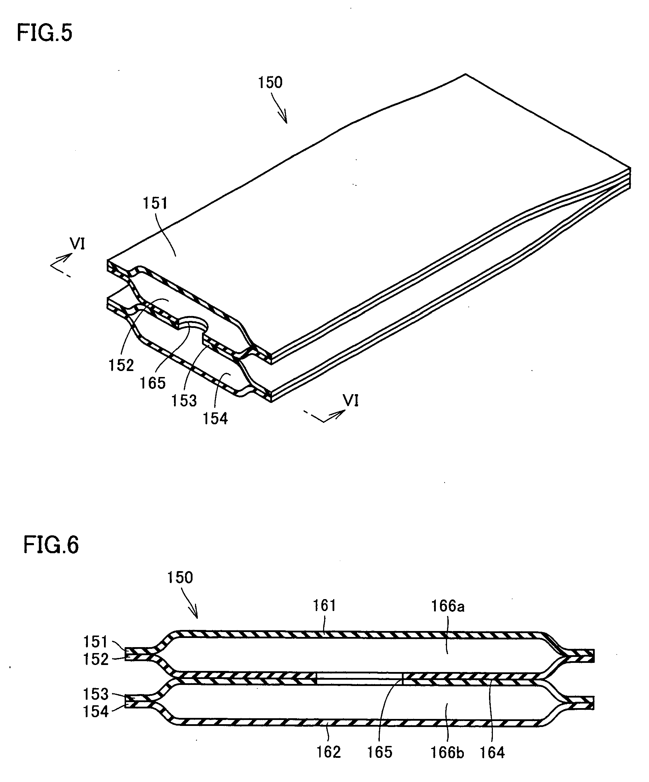

Fig. 5 is a perspective view, partially broken away, showing the air bag in this embodiment. Fig. 6 is a cross sectional view taken along line VI-VI viewed in the direction of arrows in Fig. 5, showing the air bag. Air bag 150 of the blood pressure monitor cuff in accordance with this embodiment is shaped like a bag using four resin sheets 151, 152, 153, 154. More specifically, two resin sheets 151, 152 each two-dimensionally shaped like an approximate rectangle are stacked, and the outer peripheries thereof are welded together to form a first bag body having a first inflation/deflation space 166a therein. Additionally, two resin sheets 153, 154 each two-dimensionally shaped like an approximate rectangle are stacked, and the peripheries thereof are welded together to form a second bag body having a second inflation/deflation space 166b therein. The first bag body and the second bag body are stacked with a prescribed portion welded together, so that a two-layered, integrated bag body is formed having first inflation/deflation space 166a and second inflation/deflation space 166b. It is noted that through-holes respectively corresponding to prescribed portions of two resin sheets 152, 153 of the four resin sheets are provided which are positioned at a connection portion between the first bag body and the second bag body. The through-holes serve as a communication hole 165 allowing communication between first inflation/deflation space 166a and second inflation/deflation space 166b after formation of air bag 150.

Resin sheet 154 serves as an inner circumferential sheet 162 positioned on the inner circumferential side when blood pressure monitor cuff 130 is fitted on the wrist. On the other hand, resin sheet 151 serves as an outer circumferential sheet 161 positioned on the outer circumferential side when blood pressure monitor cuff 130 is fitted on the wrist. Resin sheets 152 and 153 serve as an intermediate sheet 164.

Desirably, the material of the resin sheet forming air bag 150 is highly stretchy and free from leakage of air from inflation/deflation space 157 after welding. Such a material includes, for example, ethylene vinyl acetate copolymer (EVA), flexible polyvinyl chloride (PVC), polyurethane (PU), thermoplastic elastomer-olefinic (TPE-O), and crude rubber. Here, the present invention is characterized by air bag 150 formed of a sheet material thinner than the conventional one. Therefore, the sheet material needs to have a sufficient strength even if the thickness is reduced. In this respect, the inventors have found through an elaborate study that polyurethane is the most preferable sheet material for air bag 150. The use of a resin sheet made of polyurethane ensures a sufficient strength particularly at the welding portion.

As described above, the present invention is made based on a completely new knowledge over prior arts in that air bag 150 is formed of a sheet material thinner than the conventionally used sheet material. Accordingly, the difference in circumferential length between outer circumferential sheet 161 and inner circumferential sheet 162 is reduced when blood pressure monitor cuff 130 is wrapped around a living body, thereby decreasing big deep wrinkles formed on inner circumferential sheet 162.

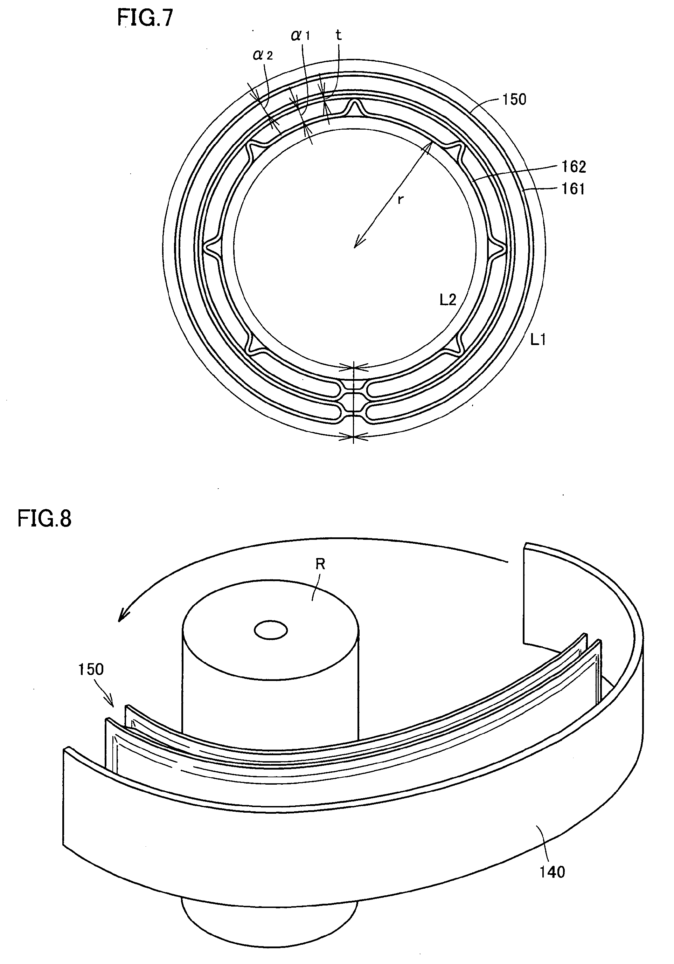

This knowledge will be described in detail. Fig. 7 illustrates each dimension of the air bag for use in calculation for verifying the knowledge. It is assumed that the upper arm or wrist on which the blood pressure monitor cuff is fitted is circular. As shown in Fig. 7, the radius of the fitting portion is r, the thickness of the sheet material forming air bag 150 is t, the total thickness of the air layers with air injected in air bag 150 is $\alpha$ (in Fig. 7, $\alpha = \alpha_1 + \alpha_2$), the circumferential length of inner circumferential sheet 162 of air bag 150 in a fitted state is L2, the circumferential length of outer circumferential sheet 161 is L1, and the number of sheets forming air bag 150 is k. Then, L1 and L2 can be expressed as follows.

$$L1 = 2\pi\,(r+kt+\alpha)$$

$$L2 = 2\pi r$$

Here, if the circumferential difference between the outer circumferential sheet and the inner circumferential sheet is Ld, Ld can be expressed as follows.

$$Ld = 2\pi\,(kt+\alpha)$$

Assuming the thickness $\alpha$ of the air layers is constant, the expression above suggests that the circumferential difference Ld can be reduced by decreasing the thickness t of the sheet material or the number of sheets k.

Here, when a plurality of air layers are provided, the thickness of air bag 150 becomes uniform in the width direction when air bag 150 is inflated, as compared with a single air layer. Therefore, a squeezing force is less likely to be distributed, so that arteries 51, 52 can be squeezed more reliably. In this respect, it is preferable to provide a plurality of air layers. When two air layers are provided, the number of sheets k is four. Therefore, as compared with a single layer (k=2), the effect brought by reducing the thickness t of the sheet material is even more remarkable.

In the following, the effect of the present invention will be verified by calculating Ld in an illustrative example. The thickness t of a sheet material forming an air bag that is most frequently used at present is in the range from 0.3 mm to 0.5 mm. It is thus assumed that the thickness t of the conventional sheet material is 0.4 mm, and the thickness t of the sheet material in the example of the present invention is 0.15 mm. With two air layers and $\alpha=0$, the circumferential difference $Ld_{0.4}$ and $Ld_{0.15}$ with t=0.4 and t=0.15 are as follows.

$$Ld_{0.4} = 2\pi\,(4{\times}0.4) = 10.1 \text{ mm}$$

$$Ld_{0.15} = 2\pi\,(4{\times}0.15) = 3.0 \text{ mm}$$

This calculation result shows that there is a very big difference between $Ld_{0.4}$ and $Ld_{0.15}$. As wrinkles are formed depending on the magnitude of Ld, reduction in the thickness of the sheet material can significantly minimize slacks on inner circumferential sheet 162, thereby reducing big deep wrinkles appearing on inner circumferential sheet 162.

Furthermore, the following experiment was conducted in order to verify this knowledge. The following three kinds of sheet materials of highly compressible PU were prepared as sheet materials forming air bag 150: a material of stretch PU having a thickness t of 0.2 mm; a material of stretch PU having a thickness t of 0.15 mm; and a material of stretch PU having a thickness t of 0.1 mm. Air bag 150 having two air layers was fabricated using each of these materials.

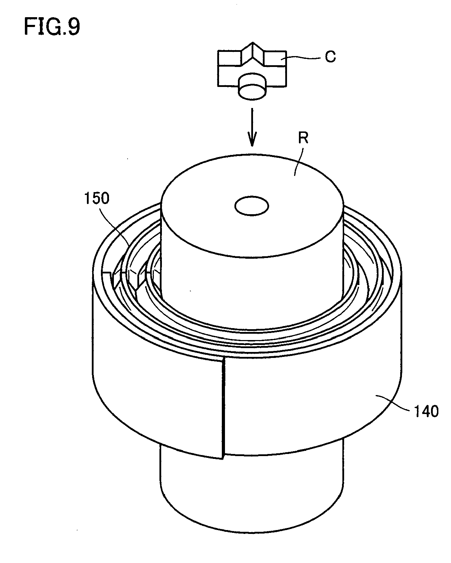

Figs. 8 and 9 illustrate the experimental method for verifying the effect of the present invention. Each of these three kinds of air bags 150 was wrapped around the outer circumference of a transparent silicon tube R modeled after a human wrist. The outer side was restrained by a belt-like cover body 140. Then, pressure was applied to air bag 150 up to about 150 mmHg, which approximately corresponds to a cuff pressure at the normal blood pressure measurement. In this state, as shown in Fig. 9, the wrinkles on the inner circumferential sheet were photographed by a photographic camera C through the end surface of transparent silicon tube R in the direction shown by the arrow.

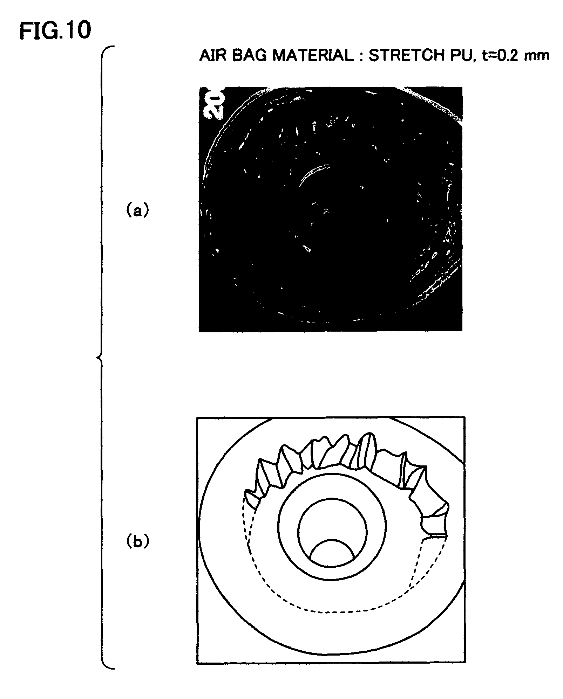

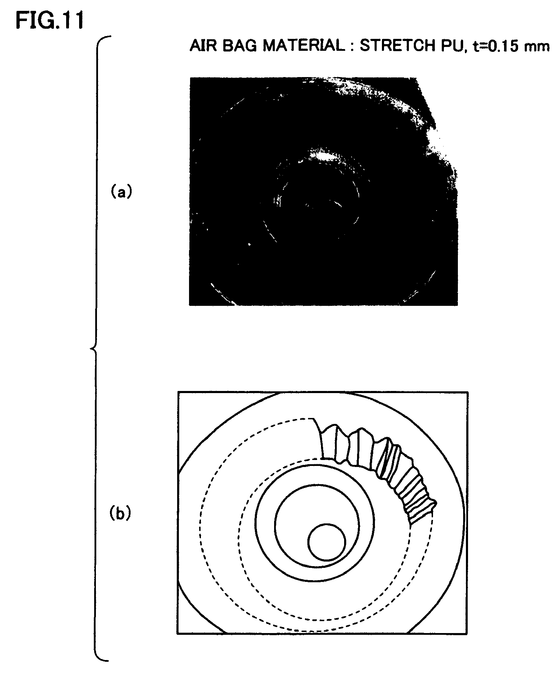

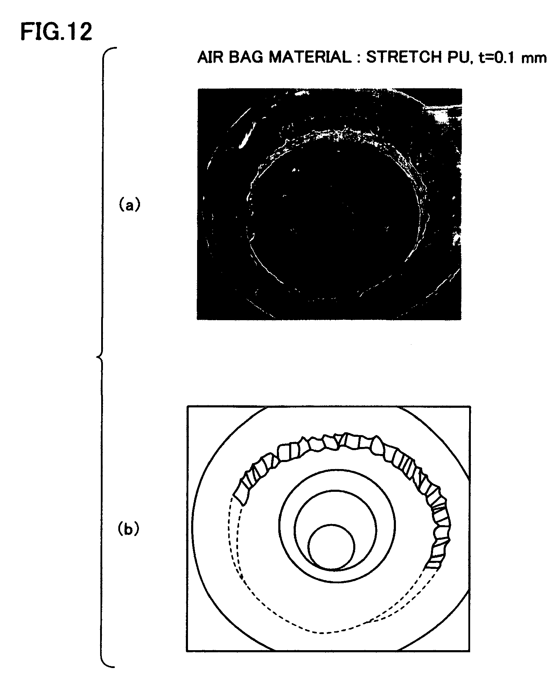

Figs. 10 to 12 show the results of this experiment, in which (a) is a photograph of the inner circumferential surface of the air bag, and (b) is an illustration based on the photograph, showing the wrinkles on the inner circumferential surface of the air bag. FIG. 10 shows a comparative example with a thickness t of 0.2 mm, Fig. 11 shows an example with a thickness t of 0.15 mm, and Fig. 12 shows an example with a thickness t of 0.1 mm. As is clear from these photographs and illustrations, with thickness t of 0.2 mm, while the wrinkles are relatively few in number, each wrinkle is very big and deep. By contrast, with thicknesses t of 0.15 mm and 0.1 mm, while the wrinkles are slightly more in number as compared with thickness of 0.2 mm, these wrinkles are extremely small and shallow.

As described above, the trouble in squeezing an artery is a deep big wrinkle (see Fig. 14). When a wrinkle is made small and shallow with thickness t of 0.15 mm or 0.1 mm, arteries can be squeezed evenly, thereby avoiding any inconvenience in blood pressure measurement caused by wrinkles.

Fig. 13 is a cross sectional view showing that arteries are squeezed using an air bag with small and shallow wrinkles appearing on the inner circumferential surface. In this figure, a single air layer is provided. As is clear from this figure, the wrinkles appearing on the inner circumferential surface of air bag 150 are extremely small and shallow, so that arteries 51, 52 can be squeezed reliably without any effect of wrinkles. In addition, since the wrinkles are extremely shallow, the wrinkles do not interrupt the passage of air in air bag 150, and air bag 150 can be inflated evenly along the entire length.

This experiment shows that thickness t of 0.15 mm or less mostly prevents big deep wrinkles. In order to prevent big deep wrinkles more reliably, preferably, thickness t of the sheet material may further be reduced. Although a thinner sheet material is preferable to reduce big deep wrinkles, considering the strength of the sheet material, it is particularly preferable that thickness t is 0.08 mm or more and 0.12 mm or less. With thickness t of less than 0.08 mm, the air bag may be broken, for example, when a great force that is not normally exerted is applied to the air bag. On the other hand, with thickness t of more than 0.12 mm, the effect of reducing big deep wrinkles may be insufficient, depending on the conditions of blood pressure measurement.

It is noted that, considering the strength of the presently available sheet material, actually, it is difficult to secure the strength of air bag 150 formed by welding unless the thickness is about 0.05 mm or more. Therefore, the minimum value of the thickness of the sheet material is 0.05 mm, if specified. It should be noted that this value may possibly be further reduced if a sheet material having a superior characteristic were found.

In this embodiment, the case of two air layers has mainly been described. However, needless to say, the present invention may be applied to the cases of a single air layer and three or more air layers. Furthermore, in the embodiment described above, the air bag is formed by welding a pair of sheet materials together at the peripheries in the shape of a bag by way of illustration. Alternatively, the air bag may be formed by folding one sheet of sheet material into half and welding the three sides together.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A blood pressure monitor cuff (130) comprising a fluid bag (150) being inflated and deflated as a fluid comes in and out, wherein
said fluid bag (150) is formed of a flexible sheet material and has at least an inner circumferential sheet (162) positioned inside when the blood pressure monitor cuff (130) is wrapped around a living body and an outer circumferential sheet (161) positioned on an outer circumferential side of the inner circumferential sheet (162), and
the sheet material forming said fluid bag (150) has a thickness of at most 0.15 mm.

2. The blood pressure monitor cuff according to claim 1, wherein said fluid bag (150) has a plurality of fluid layers (166a, 166b) radially overlapping with each other when the blood pressure monitor cuff (130) is wrapped around a living body.

3. The blood pressure monitor cuff according to claim 1, wherein the sheet material forming said fluid bag (150) is made of polyurethane.

4. The blood pressure monitor cuff according to claim 1, wherein the blood pressure monitor cuff (130) is fitted on a wrist of a human body.

5. A blood pressure monitor comprising:

the blood pressure monitor cuff(130) according to claim 1;
an inflation/deflation unit (123, 124) for inflating and deflating said fluid bag;
a pressure detection unit (122) detecting a pressure in said fluid bag; and
a blood pressure value calculation unit (113) calculating a blood pressure value based on information of the pressure detected by said pressure detection unit (122).

FIG.1

FIG.2

# FIG.3

EP 1 693 001 A1

FIG.4

```
        ┌─────────────┐
        │    START    │
        └─────────────┘
               │          ╭S101
               ▼
     ┌───────────────────┐
     │                   │
     │   INITIALIZATION  │
     │                   │
     └───────────────────┘
               │          ╭S102
               ▼
     ┌───────────────────┐
     │    APPLY CUFF     │
     │    PRESSURE       │
     └───────────────────┘
               │          ╭S103
               ▼
     ┌───────────────────┐
     │   REDUCE CUFF     │
     │    PRESSURE       │
     └───────────────────┘
               │          ╭S104
               ▼
     ┌───────────────────┐
     │   CALCULATE       │
     │   BLOOD           │
     │   PRESSURE        │
     └───────────────────┘
               │          ╭S105
               ▼
     ┌───────────────────┐
     │  DISPLAY BLOOD    │
     │  PRESSURE         │
     └───────────────────┘
               │
               ▼
        ┌─────────────┐
        │     END     │
        └─────────────┘
```

## FIG.5

## FIG.6

FIG.7

FIG.8

FIG.9

**FIG.10**

AIR BAG MATERIAL : STRETCH PU, t=0.2 mm

(a)

(b)

FIG.11

AIR BAG MATERIAL : STRETCH PU, t=0.15 mm

(a)

(b)

# FIG.12

AIR BAG MATERIAL : STRETCH PU, t=0.1 mm

(a)

(b)

## FIG.13

## FIG.14

**European Patent Office**

**EUROPEAN SEARCH REPORT**

Application Number

EP 06 00 2570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATENT ABSTRACTS OF JAPAN vol. 2002, no. 12, 12 December 2002 (2002-12-12) & JP 2002 224057 A (FUJIKURA RUBBER LTD), 13 August 2002 (2002-08-13) | 1,3-5 | INV. A61B5/022 |
| Y | * abstract * * paragraphs [0001], [0002], [0009], [0010], [0012] - [0015] * | 2 | |
| Y | PATENT ABSTRACTS OF JAPAN vol. 2000, no. 02, 29 February 2000 (2000-02-29) & JP 11 313805 A (OMRON CORP), 16 November 1999 (1999-11-16) * abstract * * paragraph [0007]; figures 1,2 * | 2 | |
| A,D | JP 62 072315 A (MATSUSHITA ELECTRIC WORKS LTD) 2 April 1987 (1987-04-02) * abstract * | 1-5 | |
| A,P | US 2005/182331 A1 (MILLAY JACK ET AL) 18 August 2005 (2005-08-18) * abstract * | 1-5 | TECHNICAL FIELDS SEARCHED (IPC) A61B |

The present search report has been drawn up for all claims

| Place of search | Date of completion of the search | Examiner |
|---|---|---|
| Munich | 17 July 2006 | Pereda Cubián, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 00 2570

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2006

| Patent document cited in search report | | Publication date | Patent family member(s) | | Publication date |
|---|---|---|---|---|---|
| JP 2002224057 | A | 13-08-2002 | NONE | | |
| JP 11313805 | A | 16-11-1999 | NONE | | |
| JP 62072315 | A | 02-04-1987 | JP<br>JP | 1615120 C<br>2042495 B | 30-08-1991<br>25-09-1990 |
| US 2005182331 | A1 | 18-08-2005 | US | 2005171445 A1 | 04-08-2005 |

EPO FORM P0459

For more details about this annex : see Official Journal of the European Patent Office, No. 12/82